# EUROPEAN PATENT SPECIFICATION

(11) **EP 3 632 482 B1**
(45) Date of publication and mention of the grant of the patent: **01.07.2026**
(21) Application number: 19209979.4
(22) Date of filing: 13.03.2014
(51) Int. Cl.: A61L 31/10, A61L 31/14

(54) **POLYMER SYSTEM FOR SECURING IMPLANTS IN SYRINGE NEEDLES**
POLYMERSYSTEM ZUM BEFESTIGEN VON IMPLANTATEN IN SPRITZENNADELN
SYSTÈME EN POLYMÈRE POUR FIXER DES IMPLANTS DANS DES AIGUILLES DE SERINGUE

(30) Priority: 14.03.2013 US 201361784502 P; 15.03.2013 US 201361789313 P
(43) Date of publication of application: 08.04.2020
(62) Divisional of application: 14714136.0
(73) Proprietor: Allergan, Inc., North Chicago, IL 60064 (US)
(72) Inventor: Spada, Lon T., Walnut, CA 91789 (US); Ghebremeskel, Alazar N., Irvine, CA 92612 (US); Robinson, Michael R., Irvine, CA 92606 (US)
(74) Representative: Hoffmann Eitle

(56) References cited:
- WO-A2-2008/019265
- WO-A2-96/07397
- US-A1- 2011 166 448

## Description

### BACKGROUND

### Field

The disclosure herein related to methods of using polymer systems for securing intraocular implants within a syringe needle.

### Description of the Related Art

Implants, such as ocular implants, are often administered to a patient through an implant administration device, such as a syringe needle. Implants, once inserted into the implant administration device, have the potential to fall out of the needle due to stresses from handling of the implant administration device with the implant inside. A hard plug may be used to secure an ocular implant within the needle. However, hard plug systems for securing an implant within a needle can damage the implant or weaken the needle.

WO2008/019265 discloses ocular implant delivery assemblies which include a cannula having a lumen extending therethrough, a proximal end, a proximal end opening, a distal end, a distal end opening, and a lumen extending through the cannula. A removable distal closure element is provided for closing the distal end opening, and a removable proximal closure element is provided for closing the proximal end opening. An ocular implant is located in the lumen. The implant may be sealed in the cannula without the addition of a liquid carrier or it may be contained in a liquid carrier medium in the cannula.

### SUMMARY

Disclosed herein are intraocular implants for use in methods of delivering intraocular implants to a target organ (eye) with a syringe needle, where the syringe needle includes a polymer retainer. Methods of making polymer retainers are also disclosed herewith. Methods of securing an intraocular implant within a syringe needle using a polymer retainer are disclosed and claimed herewith.

According to the invention, a method for securing an intraocular implant within a syringe needle includes providing a syringe needle comprising an aperture, providing a polymer, providing an intraocular implant, inserting the intraocular implant within the syringe needle, so that the intraocular implant is contained within the syringe needle, and plugging the aperture of the syringe needle with the polymer to form a polymer retainer, thus securing the intraocular implant within the syringe needle. The syringe needle includes a sharp distal tip. In some embodiments, the polymer is hydroxypropyl methyl cellulose ("HPMC"). The sharp distal tip is not coated with the polymer. In some embodiments, the syringe needle has a size selected from 0.7 mm [22 gauge], 0.5 mm [25 gauge], 0.4 mm [27 gauge], or 0.36 mm [28 gauge]. The implant is an intraocular implant. According to an embodiment, the polymer retainer increases the amount of actuation force necessary to expel the intraocular implant secured within the syringe needle by about 1% to about 25%, compared to actuation force necessary to expel the intraocular implant without the polymer retainer present.

According to another embodiment, an intraocular implant for use in a method for delivering an intraocular implant within a syringe needle to a patient in need thereof is disclosed herewith, the method includes providing a syringe needle comprising an aperture, providing a polymer comprising HPMC, providing an intraocular implant, inserting the intraocular implant within the syringe needle, so that the intraocular implant is contained within the syringe needle, plugging the aperture of the syringe needle with the polymer to form a polymer retainer, thus securing the intraocular implant within the syringe needle, inserting the syringe needle containing the intraocular implant and the polymer retainer into an organ of a patient, and administering the intraocular implant into the organ of the patient. The syringe needle includes a sharp distal tip. In other embodiments, the polymer is provided in a solution or gel form. The sharp distal tip is not coated with the polymer. In some embodiments, the syringe needle has a size selected from 0.7 mm [22 gauge], 0.5 mm [25 gauge], 0.4 mm [27 gauge], or 0.36 mm [28 gauge]. The implant is an intraocular implant. According to an embodiment, the polymer retainer increases the amount of actuation force necessary to expel the intraocular implant secured within the syringe needle by about 1% to about 25%, compared to actuation force necessary to expel the intraocular implant without the polymer retainer present.

### BRIEF DESCRIPTION OF THE DRAWINGS

These and other features will now be described with reference to the drawings summarized below. These drawings and the associated description are provided to illustrate one or more embodiments.
Figure 1 illustrates a front view of an example syringe, according to an embodiment.
Figure 2A illustrates an enlarged side view of a syringe needle coated with a polymer retainer, according to an embodiment (not according to the invention).
Figure 2B illustrates an enlarged top side view of a syringe needle plugged with a polymer retainer, according to the invention.
Figure 3 illustrates a graph comparing the penetration force of 25G needles with and without example polymer retainers.
Figure 4 shows a graph comparing the actuation force of 25G needles with and without example polymer retainers.

### DETAILED DESCRIPTION

An improved method of delivering polymer implants to an eye, includes inserting an intraocular implant into a syringe needle, then applying a polymer retainer to the distal end of the needle loaded with the intraocular implant. The polymer retainer can effectively retain the intraocular implant within the syringe needle, without significantly increasing the ejection force of the intraocular implant. In some embodiments, the polymer retainer can effectively retain the intraocular implant within a syringe needle without reducing the sharpness of the sharp distal tip of the syringe needle. The polymer retainer can advantageously be robust enough to retain the intraocular implant within the syringe needle during routine handling of the device, and still allow the intraocular implant to be ejected from the device without damaging the intraocular implant.

According to some embodiments, the polymer retainer comprises, consists of, or consists essentially of a polymer. In some embodiments, only a single polymer is used. In other embodiments, the polymer retainer comprises, consists essentially of or consists of a mixture of two or more polymers. According to some embodiments, the polymer is a cellulose ether, such as hydroxypropyl methyl cellulose "HPMC". HPMC is commercially available from the Dow Chemical Company under the brand name METHOCEL^{®}. According to other embodiments, the polymer can be hydroxypropyl cellulose, methyl cellulose, hyaluronic acid, polyvidone or povidone, carboxymethyl cellulose, polyethylene oxide, polypropylene oxide, chitosan, agarose, polypeptide, ficoll, or natural and synthetic protein. The polymer retainer may advantageously form viscous solutions, have reasonable adhesion to metals, be water soluble, and biocompatible. The specification sheets, data sheets, and testing data of these polymers are herein incorporated by reference in their entirety.

The polymer may have a suitable molecular weight. **In** some embodiments, the molecular weight can be 10,000-200,000 daltons.

The polymer may have a suitable aqueous solubility. According to some embodiments, the polymer's solubility in water can be complete. **In** some embodiments, the aqueous solubility of the polymer can be 40-60 mg/mL.

The polymer may have a suitable glass transition temperature. **In** some embodiments, the glass transition temperature of the polymer can be in the range of 150°C - 160 °C.

The polymer may have a suitable viscosity. According to some embodiments, the viscosity is in the range of 80000 cps to 120000 cps.

Suitable polymeric materials or compositions for use include those materials which are compatible, that is biocompatible, with the eye so as to cause no substantial interference with the functioning or physiology of the eye. Such materials preferably are at least partially and more preferably substantially completely biodegradable or bioerodible.

According to some embodiments, the polymer retainer can be formulated into a polymer retainer solution or polymer retainer gel before it is applied to a syringe needle. The polymer retainer solution or polymer retainer gel can contain the polymer as well as additional excipients for suitable purposes. **In** an embodiment, certain excipients may be added to the polymer retainer gel or the polymer retainer solution to modify the viscosity of the solution or gel, so that they can be easily applied to the syringe needle. According to an embodiment, an excipient comprising, consisting essentially of, or consisting of isopropyl alcohol and/or water and/or a buffer can be added to the polymer to form the polymer retainer solution or the polymer retainer gel. When used, one or more excipient can be present in the solution or gel in an amount in the range of 0.2% to 10% by weight of the solution or gel, based on the total weight of the solution or gel.

The implant administration device is a device configured to deliver an implant to an organ of a patient in need thereof.

According to some embodiments, the intraocular implant may be properly sized to fit within the syringe needle. The intraocular implant may have a diameter of about 150 µm to about 500 µm.

The implant administration device is a syringe needle. A syringe needle may have a sharp, pointed distal end configured to pierce an organ, such as the skin or eye, of a human body. A syringe needle is typically hollow, and can have a needle tip aperture, located near the sharp, pointed distal end of the syringe needle, where substances and bodies, such as an intraocular implant, may be sucked in to the needle or expelled out of the needle.

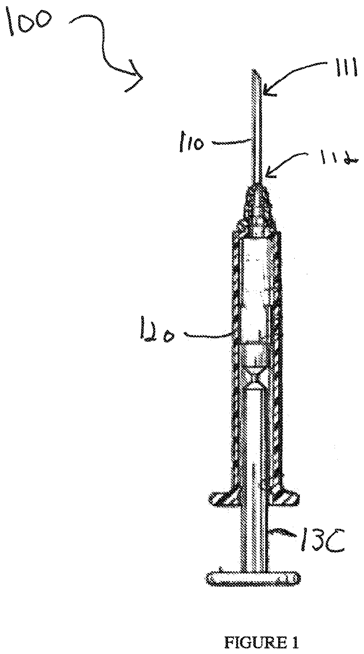

An example syringe needle 100 is illustrated in Figures 1-2B. As shown in Figure 1, syringe needle 100 includes hollow needle 110, syringe body 120, and plunger 130. As shown in Figures 1-2B, hollow needle 110, has a distal end 111, a proximal end 112, and an aperture 113. The distal end 111 has a tip portion 114 which extends from an area just proximal the aperture 115 to a sharp distal tip 116 and includes the aperture 113.

A syringe including an appropriately sized needle, for example, a 0.7 mm [22 gauge] needle, a 0.5 mm [25 gauge] needle, a 0.4 mm [27 gauge] needle, a 0.36 mm [28 gauge] needle, or a 0.31 mm [30 gauge] needle, can be effectively used to inject an implant into an organ, such an eye of a human or animal.

An intraocular implant can include a device or element that is configured to be placed in an organ, including those in the ocular region of the eye. Examples include extruded filaments, comprising a biodegradable polymer matrix and an active pharmaceutical ingredient associated with the polymer matrix, and cut to a length suitable for placement in an organ, such as an eye. Implants are generally biocompatible with physiological conditions the body (including, for example, the eye of an animal or human) and do not cause adverse reactions. In some embodiments, implant may be configured for placement in specific areas on an organ, such as the vitreous, anterior chamber, subconjunctival space or sub-tenon space of the eye. Implants, specifically intraocular implants, can be generally biocompatible with physiological conditions of an eye and do not cause adverse side effects. Implants can be biodegradable and may be produced by a suitable process, such as extrusion.

According to some embodiments, an intraocular implant may be properly sized to fit within the syringe needle.

The polymer retainer can be applied to the syringe needle, as a solution, gel, or hot melt either by dipping the needle tip into solution or by direct application of the polymer retainer to the tip of needle. In embodiments where the polymer retainer is applied to the needle tip as a solution or gel, the solution or gel solvent can be removed before the needle containing the polymer retainer is inserted into a patient. In embodiments where the polymer retainer is applied as a hot melt, the melt can be cooled after application to the needle, but before the needle containing the polymer retainer is inserted into a patient.

According to some embodiments, the entire needle is not dipped in the polymer retainer. According to some embodiment, the entire length of the needle is not coated with the polymer retainer.

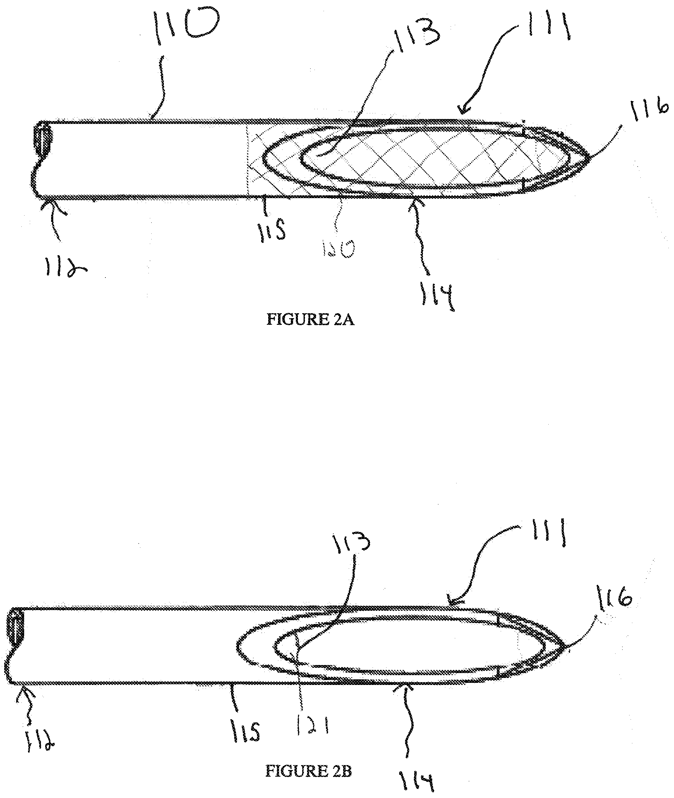

Figures 2A and 2B illustrate example embodiments of syringe needle, using polymer retainers. As illustrated in Figure 2A (not according to the invention), the distal end of the needle 111 is coated with polymer retainer 120. As shown by hatching, the polymer retainer 120 coats the tip portion 114 of the distal end 111, and the polymer retainer extends from an area just proximal the aperture 115 to a sharp distal tip 116 and includes the aperture 113. As shown in the embodiment illustrated in Figure 2B, a polymer retainer 113 is a drop or plug in the aperture 113. In the embodiment illustrated in Figure 2B (according to the invention), the sharp distal tip 116 is not coated with the polymer retainer.

The polymer retainer is administered in the aperture of the syringe needle as a plug, a suitable amount of polymer retainer may be used to effectively plug the aperture to contain the intraocular implant within the needle, but still beneficially minimize the amount of actuation force necessary to expel the intraocular implant from the needle.

In some embodiments, the needle containing a polymer retainer can be used for injections into the ocular region of a patient. The ocular region of a patient may include areas of the patient such as the patient's eyeball and surrounding areas. In some embodiments, the needle containing a polymer retainer can be used for intravitreal, intracameral, or periocular injections.

An improved method of delivering polymer implants to a target organ, such as an eye, includes inserting an intraocular implant within a syringe needle, then applying a polymer retainer to the distal end of the needle loaded with the intraocular implant.

According to some embodiments, the polymer retainer does not increase the ejection force of the implant of the needle. In some embodiments, the polymer retainer only slightly increases the amount of actuation force necessary to eject an intraocular implant contained within a syringe needle. According to some embodiments, the polymer retainer only increases the actuation force necessary to expel an intraocular implant contained within a syringe needle by about 1% to about 25%, by about 2% to about 15%, from about 1% to about 10%, from about 1% to about 5%, and the like.

According to some embodiments the polymer retainer does not reduce or only slightly reduces the sharpness of a needle tip. Thus accordingly, in some embodiments, the polymer retainer does not significantly increase the penetration force of the needle necessary to pierce an organ (such as an eye or the skin) of an animal or human. In some embodiments, the polymer retainer only slightly increases the amount of penetration force necessary to pierce an organ (such as an eye or the skin) of an animal or human. According to some embodiments, the polymer retainer only increases the amount of penetration force necessary to pierce an organ by about 1% to about 25%, by about 2% to about 15%, from about 1% to about 10%, from about 1% to about 5%, and the like.

### EXAMPLES

The polymer retainers and methods for applying the polymer retainers to a syringe needle disclosed will now be described by example embodiments below.

### EXAMPLE 1

### Manufacturing Method

In an embodiment, example polymer retainers were formulated and applied to the distal end of Hart 0.5 mm [25 G] XTW syringe needles. In a laminar flow hood, 0.25 g HPMC and 9.75 g sterile filtered 70% isopropyl alcohol were added into a Nalgene^{®} plastic sterile bottle and mixed until the HPMC was fully dissolved and a gel was formed.

Two methods were then used to add the polymer retainer to the needles. In a first embodiment, the distal end of the needles were dipped into the gel, allowing the gel fluid to flow into the distal aperture of the needles and into the hollow interior of the needle through capillary action (the comparative method, not according to the claims). In a second embodiment, a controlled dispensing system was used to directly deposit the gel onto the distal end of the needle and into the aperture of the needle.

### EXAMPLE 2

### Comparison of Penetration Force

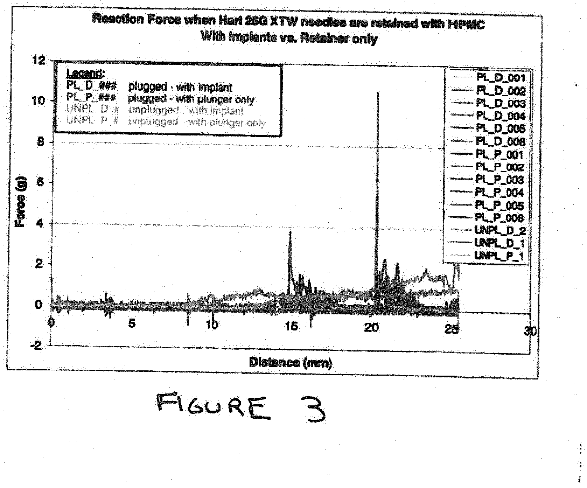

The effect of example embodiment polymer retainers on penetration force was determined by measuring the force required to penetrate a polymer membrane. Penetration force for Hart 0.5 mm [25 G] extra thin wall (XTW) needles was determined using a Texture Analyzer, Stable Micro Systems model TAXT2iHR. A 5 x 0.38 mm implant containing 35% active drug substance and 65% polymer excipients was used as the implant. Penetration force for the polymer retained needles with and without implants is shown in Figure 3. The results show that, surprisingly, both of the methods employed to apply the polymer retainer to the needle tip had no significant effects on the penetration force. A detailed visual inspection of the needle tips showed no significant effect on the needle's sharpness after applying the polymer by either method.

### EXAMPLE 3

### Determination of Ejection Force

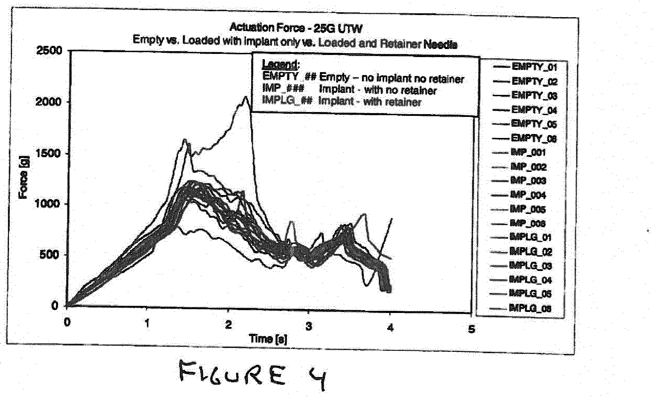

The effect of the polymer retainer on ejection force was determined by measuring the force required to eject a polymer implant from a 0.5 mm 25 [G] syringe needle. Ejection force for Hart 0.5 mm 25 [G] XTW needles was determined using a Texture Analyzer, Stable Micro Systems model TAXT2iHR. A 5 x 0.38 mm implant containing 35% active drug substance and 65% polymer excipients was used for the tests as the implant. Actuation force for the applicators with and without polymer retained implants are shown in Figure 4. The results show, surprisingly, that the polymer retainer does not require significant additional actuation force.

## Claims

1. A method for securing an intraocular implant within a syringe needle comprising:
providing a syringe needle comprising an aperture;
providing a polymer;
providing an intraocular implant;
inserting the intraocular implant within the syringe needle, so that the intraocular implant is contained within the syringe needle; and
plugging the aperture of the syringe needle with the polymer to form a polymer retainer, thus securing the intraocular implant within the syringe needle;
wherein the syringe needle comprises a sharp distal tip and wherein the sharp distal tip is not coated with the polymer.

2. The method of Claim 1, wherein the polymer is hydroxypropyl methyl cellulose.

3. The method of Claim 1 or Claim 2, wherein the syringe needle has a size selected from the group consisting of 0.7 mm [22 gauge], 0.5 mm [25 gauge], 0.4 mm [27 gauge], or 0.36 mm [28 gauge].

4. The method of any of Claims 1 to 3, wherein the polymer retainer increases the actuation force necessary to expel the intraocular implant secured within the syringe needle by about 1% to about 25%, compared to actuation force necessary to expel the intraocular implant without the polymer retainer present.

## Patentansprüche

1. Verfahren zum Sichern eines intraokularen Implantats in einer Spritzennadel, umfassend:
Bereitstellen einer Spritzennadel mit einer Öffnung;
Bereitstellen eines Polymers;
Bereitstellen eines intraokularen Implantats;
Einführen des intraokularen Implantats in die Spritzennadel, so dass das intraokulare Implantat in der Spritzennadel enthalten ist; und
Verschließen der Öffnung der Spritzennadel mit dem Polymer, um eine Polymerhalterung zu bilden, wodurch das intraokulare Implantat in der Spritzennadel befestigt wird;
wobei die Spritzennadel eine scharfe distale Spitze aufweist und wobei die scharfe distale Spitze umfasst und wobei die scharfe distale Spitze nicht mit dem Polymer beschichtet ist.

2. Verfahren nach Anspruch 1, bei dem das Polymer Hydroxypropylmethylcellulose ist.

3. Verfahren nach Anspruch 1 oder Anspruch 2, bei dem die Spritzennadel eine Größe aufweist, ausgewählt aus der Gruppe bestehend aus 0,7 mm [22 Gauge], 0,5 mm [25 Gauge], 0,4 mm [27 Gauge] oder 0,36 mm [28 Gauge].

4. Verfahren nach mindestens einem der Ansprüche 1 bis 3, bei dem die Polymerhalterung die Betätigungskraft, die erforderlich ist, um das in der Spritzennadel befestigte Intraokularimplantat auszustoßen, um etwa 1% bis etwa 25% erhöht, verglichen mit der Betätigungskraft, die erforderlich ist, um das Intraokularimplantat auszustoßen ohne dass die Polymerhalterung vorhanden ist.

## Revendications

1. Procédé destiné à fixer un implant intraoculaire dans une aiguille de seringue comprenant :
fournir une aiguille de seringue comprenant une ouverture ;
fournir un polymère ;
fournir un implant intraoculaire ;
insérer l'implant intraoculaire dans l'aiguille de seringue, de sorte que l'implant intraoculaire soit contenu dans l'aiguille de seringue ; et
obturer l'ouverture de l'aiguille de seringue avec le polymère de manière à former un moyen de retenue en polymère, assurant ainsi la fixation de l'implant intraoculaire dans l'aiguille de seringue ;
dans lequel l'aiguille de seringue comprend une pointe distale acérée et dans lequel la pointe distale acérée n'est pas revêtue du polymère.

2. Procédé selon la revendication 1, dans lequel le polymère est de l'hydroxypropylméthylcellulose.

3. Procédé selon la revendication 1 ou la revendication 2, dans lequel l'aiguille de seringue présente une taille sélectionnée dans le groupe consistant en 0,7 mm [calibre 22], 0,5 mm [calibre 25], 0,4 mm [calibre 27], ou 0,36 mm [calibre 28].

4. Procédé selon l'une quelconque des revendications 1 à 3, dans lequel le moyen de retenue en polymère augmente la force d'actionnement nécessaire pour expulser l'implant intraoculaire fixé dans l'aiguille de seringue d'environ 1 % à environ 25 %, par comparaison avec la force d'actionnement nécessaire pour expulser l'implant intraoculaire sans que le moyen de retenue en polymère soit présent.
